# EUROPEAN PATENT SPECIFICATION

(11) **EP 3 181 140 B1**
(45) Date of publication and mention of the grant of the patent: **15.07.2020**
(21) Application number: 15833729.5
(22) Date of filing: 06.08.2015
(51) Int. Cl.: A61K 31/732, A61K 47/02, A61K 47/12, A61K 9/20, A61K 9/28

(54) **DISPERSION PREPARATION CONTAINING COLLOIDAL BISMUTH PECTIN AND PREPARATION METHOD THEREFOR**
DISPERSIONSPRÄPARAT MIT KOLLOIDALEM WISMUT-PEKTIN UND HERSTELLUNGSVERFAHREN DAFÜR
PRÉPARATION DE DISPERSION CONTENANT DE LA PECTINE DE BISMUTH COLLOÏDAL ET PROCÉDÉ DE PRÉPARATION ASSOCIÉ

(30) Priority: 17.08.2014 CN 201410402727
(43) Date of publication of application: 21.06.2017
(73) Proprietor: Shanxi Zhendong Ante Biopharmaceutical Co. Ltd., Jinzhong, Shanxi 030600 (CN)
(72) Inventor: LI, Anping, Jinzhong Shanxi 030600 (CN); SHEN, Qi, Jinzhong Shanxi 030600 (CN); ZHU, Ping, Jinzhong Shanxi 030600 (CN); QIN, Zhengguo, Jinzhong Shanxi 030600 (CN); WU, Yuexia, Jinzhong Shanxi 030600 (CN); LI, Jing, Jinzhong Shanxi 030600 (CN)
(74) Representative: Laufhütte, Dieter
(86) International application number: PCT/CN2015/086243
(87) International publication number: WO 2016/026388

(56) References cited:
- CN-A- 1 634 132
- CN-A- 1 634 132
- CN-A- 101 138 554
- CN-A- 101 138 554
- CN-A- 101 229 159
- CN-A- 104 147 041
- HAN ET AL: "Research Progress on Immediate-release Dispersible Tablets", CLINICAL JOURNAL OF TRADITIOANL CHINESE MEDICINE, CN, vol. 18, no. 4, 31 August 2006 (2006-08-31), pages 414-416, XP009500245, ISSN: 1672-7134
- HAN, YUE: 'Research Progress on Immediate-release Dispersible Tablets' CLINICAL JOURNAL OF TRADITIONAL CHINESE MEDICINE vol. 18, no. 4, 31 August 2006, pages 414 - 416, XP009500245

## Description

### BACKGROUND OF THE PRESENT INVENTION

### Field of Invention

The present invention relates to a stomach medicine preparation, and more particularly to a dispersion preparation for the stomach medicine and a preparing method therefor.

### Description of Related Arts

Colloidal bismuth pectin is a colloidal-state bismuth preparation formed by a biological macromolecular material of pectin and metallic bismuth, wherein metallic bismuth is capable of killing Helicobacter pylori, so as to improve the healing rate of peptic ulcer and decrease the recurrence rate of peptic ulcer. The colloidal bismuth pectin has excellent characteristics of colloid in acidic medium and is capable of forming gel to protect mucous membrane. Under endoscopic observation, the accumulation of colloidal bismuth pectin on surfaces of ulcers was greater than that of other membranes. Thus, it can be seen that the colloidal bismuth pectin has a good selection of adhesion, and is capable of prolonging the retention time of the drug in the stomach and increasing the concentration of drugs such as antibiotics in ulcer or inflammation tissue, which facilitates thoroughly removing Helicobacter Pylori in stomach. Furthermore, the colloidal bismuth pectin also has an effect of inhibiting activity of pepsin. With high molecular weight, the colloidal bismuth pectin is difficult to be absorbed by human body, has no adverse reactions and side effects caused by the same kind of medicine. Compared with the conventional drugs, the colloidal bismuth pectin has stronger mucosal protective effects is stronger and thus is widely applied in treatments of Gastroenterology such as gastritis and gastro-duodenal ulcer and chronic gastritis.

The conventional colloidal bismuth pectin on the market includes capsules, dry suspension, powder and granules, wherein colloidal bismuth pectin capsule is a commonly used drug for treating gastrointestinal diseases. However, the effect of capsules is slowly released in treating gastrointestinal diseases and thus is not capable of meeting the clinical requirements. While treating gastrointestinal bleeding in clinical, the drug in the capsule is often poured out, mixed with water uniformly for taking, so as to make the drug play a pharmacodynamic therapeutic effect quickly to achieve a therapeutic effect. What's more, the formulations of powder, granules and dry suspension all requires to be taken after mixing with warm water, which is less convenient for taking and carrying, and has poor compliance for the patients to take.

Dispersed tablet is capable of disintegrating rapidly in 3 minutes and dissolving out, and has characteristics of rapidly exerting therapeutic effects and high bioavailability, and thus is capable of effectively solving the problems mentioned above. In addition, the dispersed tablet is convenient to administer which solves the problem of compliance of patients.

However, it is difficult to make the colloidal bismuth pectin into dispersible tablet. A main characteristic for the colloidal bismuth pectin to be different from other drugs is a higher gastric mucosa protective effect. That's because the colloidal bismuth pectin is macromolecules and has a high viscosity and colloidal stability, and is easy to form a protective film in the stomach. It is precisely because the characteristic, during the preparation process of the colloidal bismuth pectin, the disintegration characteristic of the drug and the stability of the colloidal is difficult to balance, which makes the preparation of dispersed tablets of the colloidal bismuth pectin difficult. If the colloidal stability is ensured, the colloidal tablets are difficult to disintegrate quickly and difficult to conform with regulation of evenly dispersed; if the colloidal tablets are disintegrated quickly, the regulation of evenly dispersed is easily conformed, but the stability of the colloidal is destroyed. And once the stability is destroyed, it is difficult to form the protective film in the stomach.

A Chinese patent application ZL 200310120821.0 discloses a dispersion preparation containing colloidal bismuth pectin which mainly focus on the dispersion effect of the drug, but ignores the colloidal stability, and thus mucosal protective effect of the drug is reduced. Even so, the disintegration time of the drug is long, which is over 5 minutes. Furthermore, according to the Pharmacopoeia of People's Republic of China issued by the National Pharmacopoeia Committee of China 2015 (Draft) on March 28, 2014, hereafter Chinese Pharmacopoeia for short, the test method of dispersal uniformity is modified from a conventional shaking method to "adopting a disintegration test apparatus, and an internal diameter of the mesh of a stainless steel wire is 710µm, and all should be disintegrated within 3 minutes and passed through a screen". According to the rules mentioned above, the tablets may no longer meet the requirements.

Furthermore, document CN1634132A discloses a dispersion tablet containing colloidal bismuth pectin. From Han et al. "Research Progress on Immediate-release Dispersible Tablets", Clinical Journal of Traditional Chinese Medicine, CN, Volume 18, No. 4 and CN101138554A dispersible tablets and effervescent dispersible tablets are known. In addition to that, document CN101229159A discloses an effervescent preparation for treating diarrhea and peptic ulcer.

### SUMMARY OF THE PRESENT INVENTION

An object of the present invention is to provide a method for preparing a dispersion preparation containing colloidal bismuth pectin, in such a manner that the drug is capable of being dispersed quickly and dissolved effectively, while meeting the colloidal stability characteristic of the colloidal bismuth pectin. The dispersion preparation containing colloidal bismuth pectin provided by the present invention adopts a combination of penetration enhancer and pore-forming agent, and achieves an unexpected effect in shorten a disintegration time of the colloidal bismuth pectin. The technical indicators of the present invention are in full compliance the requirements of the test method of dispersal uniformity with the second series of Chinese Pharmacopoeia 2010, and Chinese Pharmacopoeia 2015, general rule (draft) published on March 28, 2014 by National pharmacopoeia committee.

A dispersion preparation containing colloidal bismuth pectin is provided. 1g is adopted as a unit for the preparation. Each unit of the preparation includes: 44.0 to 900.0 mg of colloidal bismuth pectin, 1.0-500 mg of a penetration enhancer, 2.0-312.5mg of a acid-source pore forming agent, 2.0-250.0mg of alkali-source pore forming agent and 1.0-400.0mg of a disintegrating agent.

In the present invention, the drug containing colloidal bismuth pectin comprises all combination drugs containing colloidal bismuth pectin, such as the colloidal bismuth pectin combination drugs disclosed in a Chinese application of 200910157986.2 and ZL 200510080105.3.

The penetration enhancer is a material capable of promoting penetration of moisture into the dispersible tablet. and may comprise at least one member of calcium sulphate dehydrate and calcium bicarbonate. According to the invention, the penetration enhancer comprises calcium sulphate dehydrate.

In the present invention, substances capable of generating bubbles serve as pore-forming agent. The pore-forming agent is cooperated by an acid resource pore-forming agent and an alkali resource pore-forming agent. The acid resource pore-forming agent is a solid material capable of being ionized into H⁺. The acid resource pore-forming agent is selected from at least one member from anhydrous citric acid, monohydrate citric acid, tartaric acid, fumaric acid, monopotassium phosphate, sodium dihydrogen phosphate, glycine and EDTA. The alkali resource pore-forming agent is sodium bicarbonate.

The disintegrating agent is at least one member selected from low substituted hydroxypropy cellulose, crospovidone, croscarmellose sodium and sodium carboxymethyl starch.

The difficulty of preparing the colloidal bismuth pectin lies in disintegration, which is caused by the nature of the drug. The colloidal bismuth pectin forms a colloidal protective layer after meeting the moisture. It is difficult for the colloidal bismuth pectin to penetrate into the dispersant from outside, so that the dispersant is difficult to disintegrate quickly.

The present invention adopts a method of adding penetration enhancer, and is capable of greatly accelerating the speed of the moisture penetrating into the dispersant. Addition of the penetration enhancer of the present invention is capable of making the dispersant rapidly expanded and disintegrate into large blob which is difficult to be disintegrated into tiny particles. Research indicates that with the increase of content of the penetrant, though the disintegrating rate of the dispersant accelerates, the dissolution of the drug shows a decreasing trend. Thus, the present invention further adopts the pore-forming agent, and adds acid-resource pore-forming agent and alkali resource pore-forming agent to generate resource pore-forming agent.

The dispersion preparation containing colloidal bismuth pectin of the present invention comprises the penetration enhancer, the pore-forming agent and the disintegrating agent and further comprises lubricant.

The lubricant is at least one member selected from glyceryl behenate, poloxamer188, sodium dodecyl sulfate, PEG6000, silicon dioxide and talcum powder. An additive amount of the lubricant is 5.0-50.0mg for each gram of the preparation

The dispersion preparation containing colloidal bismuth pectin of the present invention further comprises a filler, wherein the filler is at least one member selected from lactose, microcrystalline cellulose, mannitol, sucrose and pregelled starch. An additive amount of the filler is 1.0-375.0mg for each gram of the preparation.

Preferably, dispersion preparation containing colloidal bismuth pectin of the present invention is made into dispersion tablets.

The dispersion tablets of the present invention are prepared by a method comprising steps of: mixing a required quantity of the crude material and the auxiliary material by drying mix and tablet compressing; or by adding 160-800µl adhesive for each gram of preparation into a required quantity of the crude material and the auxiliary material, and then performing wet granulation and compression.

The adhesive is at least one member selected from a group consisting of water, 30vol%-80vol% ethyl alcohol, 30vol%-80vol% ethanol solution containing 3wt%-5wt% polyvinylpyrrolidone, and 0.2wt% tween-80 aqueous solution.

Dispersed colloidal bismuth pectin-containing formulations of the present invention provides a pharmaceutical solution difficult to contain colloidal bismuth pectin dispersion formulation prepared in line with requirements, but also to meet the dispersed colloidal bismuth pectin formulations of the drug itself features the puzzle, made of colloidal bismuth pectin dispersion formulation is consistent dispersion uniformity shall also meet the requirements of dissolution, while maintaining a stable colloidal properties colloidal bismuth pectin.

Dispersed colloidal bismuth pectin-containing formulations of the present invention can provide rapid disintegration and dissolution in 3 minutes, rapid play efficacy, shorten the onset time of the drug, can play a role in the treatment of acute gastrointestinal diseases, expanding the colloid fruit bismuth glue application market. Meanwhile, the dispersion formulations are also easier to use, carry and storage, improved patient compliance.

The method of the present invention, respectively, following the determination of the dispersion containing colloidal bismuth pectin formulations, dissolution, colloidal stability and uniformity of dispersion.
1. Determination Methods: The method for the determination of the Chinese Pharmacopoeia 2010 edition of two Page 852 ∼ 853, colloidal bismuth pectin.
2. The dissolution test: The Chinese Pharmacopoeia 2010 edition of Appendix X C dissolution test method, determination method and second pages 852 - 853 Determination of colloidal bismuth pectin.
3. Determination of the colloidal stability: according to the following Chinese Pharmacopoeia 2010 edition of two "colloidal bismuth pectin" item "colloidal stability" to develop, take colloidal bismuth pectin formulations dispersed in 100ml stoppered graduated cylinder, add water to 100ml, strong shaking for 1 minute to a colloidal solution, stand for 1 hour, the top surface of the colloidal material may not scale down to 97ml or less.
4. Uniform dispersion of the measurement method: The Chinese Pharmacopoeia 2010 edition of Appendix XA Disintegration Test Method The disintegration test apparatus, stainless steel wire mesh inner diameter of 710µm, the water temperature is 15-25°C; colloid containing fruit bismuth dispersion adhesive formulation should be fully disintegrated and through the screen within three minutes.

The present invention is dispersed colloidal bismuth pectin-containing formulations via the above-described detecting method, dissolution of the formulation is greater than 80%, in line with the requirements of the colloidal stability, uniformity of dispersion of less than 2.5 minutes.

Furthermore, the factors that affect the dispersion preparation containing colloidal bismuth pectin of the present invention are studied.

### 1. High temperature test

**Table 1**

| Batch number | Time | Appearance | Dispersal uniformity | Dissolution rate | Contents |
|---|---|---|---|---|---|
| 2014070501 | 0 day | Yellow tablet | 125s | 82.50% | 98.00% |
| 5 day | Yellow tablet | 121s | 83.60% | 99.70% | |
| 10 day | Yellow tablet | 126s | 81.50% | 96.50% | |

The test result indicates that the indicators of drug have no changes after being placed under 60°C for 10 days.

### 2. High humidity test

The dispersants of the colloidal bismuth pectin are put into an open dish and spread into a thin layer with a thickness of ≤5mm, and is precisely tested, then placed in two constant humidity and sealed ovens, wherein the humidity is respectively adjusted to be at 92.5% and 75.0% under 25°C for 10 days. Samples are taken respectively on the fifth day and the tenth days. Test according to a stable program. Meanwhile, the drug is precisely weighed respectively before and after the test, so as to understand moisture absorption and deliquescence performance of the drug, and results are shown as follows.

Table 2 Results of high humidity test (92.5%)

**Table 2**

| Batch number | Time | Appearance | Moisture-absorption weight gain | Dispersal uniformity | Dissolution rate | Contents |
|---|---|---|---|---|---|---|
| 2014070501 | 0 day | Yellow tablet | - | 125s | 82.50% | 98.00% |
| 5 day | Yellow tablet | 15.80% | 101s | 82.40% | 99.70% | |
| 10 day | Yellow tablet | 16.90% | 99s | 82.90% | 97.50% | |

Table 3 Results of high humidity test (75.0%)

**Table 3**

| Batch number | Time | Appearance | Moisture-absorption weight gain | Dispersal uniformity | Dissolution rate | Contents |
|---|---|---|---|---|---|---|
| 2014070501 | 0 day | Yellow tablet | - | 125s | 82.50% | 98.00% |
| 5 day | Yellow tablet | 13.80% | 112s | 82.40% | 99.60% | |
| 10 day | Yellow tablet | 14.90% | 106s | 83.10% | 598.40% | |

The test result indicates that placing under the temperature of 25°C and the humidity of 92.5% for 10 days, weight gained by moisture is over 5%, and the uniformity of dispersion is shortened. Other quality indicators remain basically unchanged.

### 3. High light exposure test

The dispersants of the colloidal bismuth pectin are put into an open dish and spread into a thin layer with a thickness of ≤5mm, then disposed in a adjustable dimmer box with an illuminance of 4500lx for 10 days, Samples are taken respectively on the fifth day and the tenth days. Test according to a stable program, special attention is paid on the appearances of the drugs, and results are shown as follows.

Table 4 Results of high light exposure test

**Table 4**

| Batch number | Time | Appearance | Dispersal uniformity | Dissolution rate | Contents |
|---|---|---|---|---|---|
| 2014070501 | 0 day | Yellow tablet | 125s | 82.50% | 98.00% |
| 5 day | Yellow tablet | 131s | 82.20% | 101.30% | |
| 10 day | Yellow tablet | 120s | 83.40% | 99.90% | |

The test result indicates that the indicators of drug have no changes after being disposed under high light exposure for 10 days.

### DETAILED DESCRIPTION OF THE PREFERRED EMBODIMENT

Colloidal bismuth pectin adopted in preferred embodiments of the present invention comprises 14-16% (mass percent) metallic bismuth.

### Example 1

Formulation: 324.7g of colloidal bismuth pectin, 25.0g of low substituted hydroxypropy cellulose, 80.8g of microcrystalline cellulose, 15.Og of calcium sulphate dehydrate, 27.5g monohydrate citric acid, 22.0g of sodium bicarbonate, 5.0g of talcum powder, and 80.0-400.0ml ethyl alcohol with a concentration of 50%. 1000 tablets are made, and each tablet weighs 500mg.

A preparation process of the dispersion preparation comprises steps of:
grinding a crude material and an auxiliary material;
screening by a 100 mesh screen;
weighing a formula dosage of colloidal bismuth pectin, low substituted hydroxypropy cellulose, microcrystalline cellulose, calcium sulphate dehydrate and sodium bicarbonate and mixing uniformly;
adding a formula dosage of soft material made by 50% ethanol, screening by 24 mesh screen, granulating, drying for 2 hours under 60°C, and screening whole grains by 24 meshes, adding monohydrate citric acid and talcum powder, mixing uniformly; and
testing drug contents, tabletting, coating a film, testing and packaging.

Testing indicators: hardness 3.0±0.5kg, dispersal uniformity 135s, dissolution rate 80.19%, and a weight difference meets requirements.

### Example 2

Formulation:324.7g of colloidal bismuth pectin, 26.5g of low substituted hydroxypropy cellulose, 98.0g of microcrystalline cellulose, 15.9g of calcium sulphate dehydrate, 33.1g monohydrate citric acid, 26.5g of sodium bicarbonate, 5.3g of talcum powder. 1000 tablets are made, and each tablet weighs 500mg.

A preparation process of the dispersion preparation comprises steps of:
grinding a crude material and an auxiliary material;
screening by a 100 mesh screen;
weighing a formula dosage of colloidal bismuth pectin, low substituted hydroxypropy cellulose, microcrystalline cellulose, calcium sulphate dehydrate, sodium bicarbonate, anhydrous citric acid and talcum powder and mixing uniformly;
testing drug contents, tabletting, testing and packaging.

Testing indicators: hardness 3.0±0.5kg, dispersal uniformity 135s, dissolution rate 80.19%, and a weight difference meets requirements.

### Examples 3-9

**Table 5**

| Crude material and auxiliary material | Example 3 | Example 4 | Example 5 | Example 6 | Example 7 | Example 8 | Example 9 |
|---|---|---|---|---|---|---|---|
| Colloidal bismuth pectin, mg | 17.6 | 324.7 | 324.7 | 259.7 | 324.7 | 324.7 | 324.7 |
| lactose, mg | 50.0 | 25.0 | - | 25.0 | - | - | - |
| microcrystalline cellulose, mg | 103.0 | 48.7 | 80.8 | 177.2 | 7.7 | 92.0 | 18.5 |
| hydroxypropy cellulose, mg | - | 25.0 | 25.0 | 25.0 | 26.5 | 26.5 | 26.5 |
| calcium sulphate dehydrate, mg | 0.4 | 15.0 | 15.0 | 12.5 | 70.4 | 15.9 | 47.7 |
| sodium bicarbonate, mg | 100.0 | 25.0 | 22.0 | 20.0 | 42.4 | 29.2 | 47.7 |
| anhydrous citric acid, mg | 125.0 | - | 27.5 | - | - | - | 59.6 |
| monohydrate citric acid, mg | - | 31.3 | - | 25.0 | - | - | - |
| fumaric acid, mg | - | - | - | - | - | 36.4 | - |
| tartaric acid, mg | - | - | - | - | 53.0 | - | - |
| poloxamer 188, mg | - | 5.0 | - | - | - | - | - |
| sodium dodecyl sulfate, mg | 4.0 | - | - | - | - | - | - |
| PEG6000, mg | - | - | 5.0 | - | - | - | - |
| silicon dioxide, mg | - | - | - | 4.8 | - | - | - |
| magnesium stearate, mg | - | - | - | 1.0 | - | - | - |
| talcum powder, mg | - | - | - | - | 5.3 | 5.3 | 5.3 |
| 50% ethanol,µl/tablet | - | 400.0 | 400.0 | - | - | - | - |
| 3% polyvinylpyrrolid one 50% ethanol solution,µl/tablet | - | - | - | - | 100.0 | - | - |
| 0.2% tween-80,µl/tablet | 80.0 | - | - | 100.0 | - | 100.0 | 100.0 |
| tablet weight,mg | 400 | 499.7 | 500 | 550.2 | 530 | 530 | 530 |
| dispersible uniformity, s | 78 | 120 | 125 | 132 | 136 | 125 | 118 |
| dissolution rate,% | 98.8 | 81.6 | 80.2 | 85.6 | 91.6 | 85.4 | 96.8 |
| Colloidal stability | conforms with regulations | conforms with regulations | conforms with regulations | conforms with regulations | conforms with regulations | conforms with regulations | conforms with regulations |

### Examples 10-15

**Table 6**

| Crude material and auxiliary material | Example 10 | Example 11 | Example 12 | Example 13 | Example 14 | Example 15 |
|---|---|---|---|---|---|---|
| Colloidal bismuth pectin, mg | 324.7 | 974.0 | 324.7 | 73.0 | 173.7 | 211.2 |
| lactose,mg | - | 50.0 | - | - | - | 50.0 |
| microcrystalline cellulose, mg | 52.9 | 200.0 | - | 75.0 | - | 100.0 |
| hydroxypropy cellulose, mg | - | - | - | - | 25.0 | - |
| saccharose,mg | - | - | - | - | 20.0 | 37.5 |
| pregelatinized starch, mg | - | - | - | - | 5.0 | - |
| hydroxypropyl cellulose, mg | 26.5 | 65.0 | - | 25.0 | 100.0 | 25.0 |
| crospovidone, mg | - | - | - | - | 50.0 | - |
| croscarmellose sodium, mg | - | - | - | 45.0 | 50.0 | - |
| carboxyl methyl starch sodium,mg | - | - | - | 5.0 | - | - |
| calcium sulphate dihydrate, mg | 25.2 | 40.0 | 15.0 | 250.0 | 15.0 | 15.0 |
| sodium bicarbonate, mg | 42.4 | 74.0 | 15.0 | 1.0 | 25.0 | 25.0 |
| anhydrous citric acid, mg | - | 90.0 | - | 1.0 | - | 31.3 |
| monohydrate citric acid, mg | - | - | 15.0 | - | - | - |
| fumaric acid, mg | 53.0 | - | - | - | - | - |
| tartaric acid,mg | - | - | - | - | 31.3 | - |
| Glyceryl Behenate, mg | - | 15.0 | - | - | - | - |
| silicon dioxide, mg | - | - | | 25.0 | | |
| talcum powder, mg | 5.3 | | 5.0 | - | 5.0 | 5.0 |
| 50% ethanol solution, µl/tablet | - | - | 100.0 | - | - | - |
| 3% polyvinylpyrrolidone 50% ethanol solution,µl/tablet | - | 200.0 | - | - | - | - |
| 0.2% tween-80,µl/ tablet | 100.0 | - | - | 100.0 | - | - |
| tablet weight, mg | 530 | 1508 | 374.7 | 500 | 500 | 500 |
| dispersible uniformity,s | 120 | 143 | 110 | 123 | 136 | 105 |
| dissolution rate,% | 98.8 | 85.0 | 85.3 | 88.9 | 85.1 | 86.6 |
| Colloidal stability | Conforms with regulations | Conforms with regulations | Conforms with regulations | Conforms with regulations | Conforms with regulations | Conforms with regulations |

## Claims

1. A dispersion preparation containing colloidal bismuth pectin, wherein 1 g of the preparation comprises:
44.0-900.0mg of colloidal bismuth pectin;
1.0-500mg of a penetration enhancer, wherein the penetration enhancer is a material capable of promoting penetration of moisture into dispersible tablet;
2.0-312.5mg of acid-source pore forming agent;
2.0-250.0mg of alkali-source pore forming agent; and
1.0-400.0mg of a disintegrating agent;
wherein the penetration enhancer comprises calcium sulphate dihydrate;
the acid-source pore-forming agent is selected from at least one member from anhydrous citric acid, monohydrate citric acid, tartaric acid, fumaric acid, monopotassium phosphate, sodium dihydrogen phosphate, glycine and EDTA; and
the alkali-source pore-forming agent is sodium bicarbonate.

2. The dispersion preparation containing colloidal bismuth pectin, as recited in claim 1, wherein each gram of the dispersion preparation containing colloidal bismuth pectin further comprises 5.0-50.0mg lubricant, and the lubricant is at least one member selected from glyceryl behenate, poloxamer188, sodium dodecyl sulfate, PEG6000, silicon dioxide and talcum powder.

3. The dispersion preparation containing colloidal bismuth pectin, as recited in claim 1 or 2, wherein the dispersion preparation comprises filler.

4. The dispersion preparation containing colloidal bismuth pectin, as recited in claim 3, wherein an addition amount of the filler is 1.0-375.0mg in each gram of the dispersion preparation.

5. The dispersion preparation containing colloidal bismuth pectin, as recited in claim 3, wherein the filler is at least one member selected from lactose, microcrystalline cellulose, mannitol, sucrose and pregelled starch.

6. The dispersion preparation containing colloidal bismuth pectin, as recited in claim 1, wherein the disintegrating agent is at least one member selected from low substituted hydroxypropyl cellulose, crospovidone, croscarmellose sodium or sodium carboxymethyl starch.

7. The dispersion preparation containing colloidal bismuth pectin, as recited in claim 1 or 2, wherein the dispersion preparation is dispersed tablet.

8. A method for preparing the dispersion preparation containing colloidal bismuth pectin, as recited in claim 7, comprising steps of: mixing colloidal bismuth pectin, the penetration enhancer, the acid-source pore forming agent, the alkali-source pore forming agent, the disintegrating agent and optionally the lubricant by drying mix and tablet compressing.

9. A method for preparing the dispersion preparation containing colloidal bismuth pectin, as recited in claim 7, comprising steps of: adding adhesive to the colloidal bismuth pectin, the penetration enhancer, the acid-source pore forming agent, the alkali-source pore forming agent and the disintegrating agent and optionally the lubricant, pelleting by a wet process and then tablet compressing.

10. The method for preparing the dispersion preparation containing colloidal bismuth pectin, as recited in claim 9, wherein the adhesive is at least one member selected from a group consisting of water, 30vol%-80vol% ethyl alcohol, 30vol%-80vol% ethanol solution containing 3wt%-5wt% polyvinylpyrrolidone, and 0.2wt% tween-80 aqueous solution, wherein an additive amount of the adhesive is 160∼ 800µl/g for each gram of the dispersion preparation.

## Patentansprüche

1. Dispersionspräparat, das kolloidales Bismutpektin enthält, wobei 1 g des Präparats aufweist:
44,0-900,0 mg kolloidales Bismutpektin;
1,0-500 mg eines Penetrationsförderers, wobei es sich bei dem Penetrationsförderer um ein Material handelt, das in der Lage ist, ein Eindringen von Feuchtigkeit in eine dispergierbare Tablette zu fördern;
2,0-312,5 mg Porenbildner mit Säurequelle;
2,0-250,0 mg Porenbildner mit Alkaliquelle; und
1,0-400,0 mg eines Sprengmittels;
wobei der Penetrationsförderer Calciumsulfat-Dihydrat aufweist;
der Porenbildner mit Säurequelle aus wenigstens einem Element aus wasserfreier Citronensäure, Citronensäure-Monohydrat, Weinsäure, Fumarsäure, Monokaliumphosphat, Natriumdihydrogenphosphat, Glycin und EDTA ausgewählt ist; und
der Porenbildner mit Alkaliquelle Natriumhydrogencarbonat ist.

2. Dispersionspräparat, das kolloidales Bismutpektin enthält, nach Anspruch 1, wobei jedes Gramm des Dispersionspräparats, das kolloidales Bismutpektin enthält, ferner 5,0-50,0 mg Schmiermittel aufweist, und das Schmiermittel wenigstens ein Element ist, das aus Glycerylbehenat, Poloxamer 188, Natriumdodecylsulfat, PEG 6000, Siliciumdioxid und Talkum ausgewählt ist.

3. Dispersionspräparat, das kolloidales Bismutpektin enthält, nach Anspruch 1 oder 2, wobei das Dispersionspräparat einen Füllstoff aufweist.

4. Dispersionspräparat, das kolloidales Bismutpektin enthält, nach Anspruch 3, wobei eine Zusatzmenge des Füllstoffs 1,0-375,0 mg in jedem Gramm des Dispersionspräparats beträgt.

5. Dispersionspräparat, das kolloidales Bismutpektin enthält, nach Anspruch 3, wobei der Füllstoff wenigstens ein Element ist, das aus Lactose, mikrokristalliner Cellulose, Mannitol, Saccharose und vorgelierter Stärke ausgewählt ist.

6. Dispersionspräparat, das kolloidales Bismutpektin enthält, nach Anspruch 1, wobei das Sprengmittel wenigstens ein Element ist, das aus niedrig substituierter Hydroxypropylcellulose, Crospovidon, Croscarmellose-Natrium und Natriumcarboxymethylcellulose ausgewählt ist.

7. Dispersionspräparat, das kolloidales Bismutpektin enthält, nach Anspruch 1 oder 2, wobei das Dispersionspräparat eine dispergierte Tablette ist.

8. Verfahren zum Herstellen des Dispersionspräparats, das kolloidales Bismutpektin enthält, nach Anspruch 7, die folgenden Schritte aufweisend: Mischen von kolloidalem Bismutpektin, dem Penetrationsförderer, dem Porenbildner mit Säurequelle, dem Porenbildner mit Alkaliquelle, dem Sprengmittel und optional dem Schmiermittel durch Trocknen der Mischung und Pressen der Tablette.

9. Verfahren zum Herstellen des Dispersionspräparats, das kolloidales Bismutpektin enthält, nach Anspruch 7, die folgenden Schritte aufweisend: Hinzufügen eines Klebstoffs zu dem kolloidalen Bismutpektin, dem Penetrationsförderer, dem Porenbildner mit Säurequelle, dem Porenbildner mit Alkaliquelle, dem Sprengmittel und optional dem Schmiermittel, Pelletieren durch ein Nassverfahren und dann Pressen der Tablette.

10. Verfahren zum Herstellen des Dispersionspräparats, das kolloidales Bismutpektin enthält, nach Anspruch 9, wobei der Klebstoff wenigstens ein Element ist, das aus einer Gruppe ausgewählt ist, die aus Wasser, 30 Vol.-%-80 Vol.-% Ethylalkohol, 30 Vol.-%-80 Vol.-% Ethanollösung, die 3 Gew.-%-5 Gew.-% Polyvinylpyrrolidon enthält, und 0,2 Gew.-% Tween 80 in wässriger Lösung besteht, wobei eine Zusatzstoffmenge des Klebstoffs 160-∼800 µl/g für jedes Gramm des Dispersionspräparats beträgt.

## Revendications

1. Préparation de dispersion contenant de la pectine de bismuth colloïdal, dans laquelle 1 g de la préparation comprend :
44,0-900,0 mg de pectine de bismuth colloïdal ;
1,0-500 mg d'un agent améliorant la pénétration, dans laquelle l'agent améliorant la pénétration est un matériau en mesure de promouvoir la pénétration d'humidité dans un comprimé dispersible ;
2,0-312,5 mg d'un agent de formation de pores de source acide ;
2,0-250,0 mg d'un agent de formation de pores de source alcaline, et
1,0-400,0 mg d'un délitant ;
dans laquelle l'agent améliorant la pénétration comprend du sulfate de calcium dihydraté ;
l'agent de formation de pores de source acide est sélectionné parmi au moins un élément parmi l'acide citrique anhydre, l'acide citrique monohydraté, l'acide tartrique, l'acide fumarique, le phosphate monopotassique, le phosphate monosodique, la glycine, et l'EDTA (éthylène diamine tétra acétate), et
l'agent de formation de pores de source alcaline est du bicarbonate de sodium.

2. Préparation de dispersion contenant de la pectine de bismuth colloïdal selon la revendication 1, dans laquelle chaque gramme de la préparation de dispersion contenant de la pectine de bismuth colloïdal comprend en outre 5,0-50,0 mg de lubrifiant, et le lubrifiant est au moins un élément sélectionné parmi du béhénate de glycéryle, du poloxamère 188, du dodésulfate de sodium, du PEG 6000 (polyéthylène glycol), du dioxyde de silicium, et de la poudre de talc.

3. Préparation de dispersion contenant de la pectine de bismuth colloïdal selon la revendication 1 ou 2, dans laquelle la préparation de dispersion comprend une charge.

4. Préparation de dispersion contenant de la pectine de bismuth colloïdal selon la revendication 3, dans laquelle une quantité d'addition de la charge est de 1,0-375,0 mg dans chaque gramme de la préparation de dispersion.

5. Préparation de dispersion contenant de la pectine de bismuth colloïdal selon la revendication 3, dans laquelle la charge est au moins un élément sélectionné parmi le lactose, la cellulose microcristalline, le mannitol, le sucrose, ou de l'amidon prégélatinisé.

6. Préparation de dispersion contenant de la pectine de bismuth colloïdal selon la revendication 1, dans laquelle le délitant est au moins un élément sélectionné parmi l'hydroxypropyl cellulose à faible substitution, la crospovidone, la croscarmellose sodique, ou du carboxyméthylamidon sodique.

7. Préparation de dispersion contenant de la pectine de bismuth colloïdal selon la revendication 1 ou 2, dans laquelle la préparation de dispersion est un comprimé dispersible.

8. Procédé destiné à préparer la préparation de dispersion contenant de la pectine de bismuth colloïdal selon la revendication 7, comprenant les étapes consistant à : mélanger la pectine de bismuth colloïdal, l'agent améliorant la pénétration, l'agent de formation de pores de source acide, l'agent de formation de pores de source alcaline, et le délitant, et facultativement le lubrifiant, par un mélange à sec et un compactage en comprimés.

9. Procédé destiné à préparer la préparation de dispersion contenant de la pectine de bismuth colloïdal selon la revendication 7, comprenant les étapes consistant à : ajouter un adhésif à la pectine de bismuth colloïdal, l'agent améliorant la pénétration, l'agent de formation de pores de source acide, l'agent de formation de pores de source alcaline, et le délitant, et facultativement le lubrifiant, former des pastilles par un processus par voie humide, puis effectuer un compactage en comprimés.

10. Procédé destiné à préparer la préparation de dispersion contenant de la pectine de bismuth colloïdal selon la revendication 9, dans lequel : l'adhésif est au moins un élément sélectionné parmi un groupe consistant en de l'eau, de l'alcool éthylique à 30 % en volume-80 % en volume, une solution d'éthanol à 30 % en volume-80 % en volume contenant de la polyvinylpyrrolidone à 3 % en poids-5 % en poids, et une solution aqueuse à 0,2 % en poids de tween-80, dans lequel une quantité d'addition de l'adhésif est 160∼800 µl/g pour chaque gramme de la préparation de dispersion.
